# EUROPEAN PATENT APPLICATION

(11) **EP 4 756 032 A2**
(43) Date of publication of application: **10.06.2026**
(21) Application number: 25219783.5
(22) Date of filing: 01.12.2025
(51) Int. Cl.: C12P 7/6409, C12P 7/6463, C12P 7/6472, C12N 1/18, C12N 15/01, C11B 1/10

(54) **PROCESS FOR PRODUCTION OF MICROBIAL OIL**

(30) Priority: 05.12.2024 IN 202421096140
(71) Applicant: Indian Oil Corporation Limited, Mumbai, Maharashtra 400 051 (IN)
(72) Inventor: SIVAGURUNATHAN, P, Faridabad-121007, Haryana (IN); SAHOO, Prakash Chandra, Faridabad-121007, Haryana (IN); KUMAR, Manoj, Faridabad-121007, Haryana (IN); BOMPELLI, Sravan, Faridabad-121007, Haryana (IN); GUPTA, Ravi Prakash, Faridabad-121007, Haryana (IN); SRIVASTVA, Umish, Faridabad-121007, Haryana (IN)
(74) Representative: dompatent

(57) **Abstract**

The invention relates to the process for conversion of organic rich feed stock or industrial byproduct or CO₂ derived intermediates into tailor made microbial oil. This process involves the addition of growth inducers and providing intermittent temperature-shock to induce the biomass yield and oil accumulation. The mutated yeast used herein improves the synthesis trait of tailor-made chain length and process conditions herein to improve the production yield of biomass and oil quality. The mutated yeast was subject to process optimization with varied temperature-shock conditions and the presence of growth inducers. The produced microbial oil has potential application for biodiesel, biolubricants and bio-additives.

## Description

### FIELD OF THE INVENTION

The present invention relates to a process for the preparation of microbial oils having application in biodiesel, biolubricants and bio-additives. More specifically, the present invention relates to a process for the conversion of a waste feedstock into tailor made microbial oil using a mutant yeast strain and in the presence of growth inducers.

### BACKGROUND OF THE INVENTION

Microbial oil produced by oleaginous bacteria, algae, yeast, and fungi are promising sustainable platform chemicals. Microbial oil is chemically equivalent to plant oils, but can be produced independent of season, climate and location using a wide range of cheap and abundant carbon sources including waste streams from food and other agriculturally based industries or renewable carbon sources and therefore do not compete with food or feed. In particular, microbial oil production with yeasts is advantageous because of their fast growth rate and greater convenience to scale up cultivation than that of autotrophic microalgae since no light is needed. Oleaginous yeast lipids can be used to produce biodiesel and might find application in various industries (food, feed, Oil industries) and as building blocks for biopolymers, biolubricants or bio additives.

Microorganisms synthesize lipids with distinct carbon chain lengths and degrees of unsaturation. These fatty acids can be stored in organelles, termed lipid bodies or lipid droplets, as storage lipids, for example, as triacylglycerides (TAG). The lipid profile of a cell, i.e., the relative amounts of fatty acid species that make up the total lipids in the cell, is determined by the activities and substrate specificities of various enzymes that synthesize fatty acids (fatty acid synthase, elongase, desaturase), various enzymes that stabilize fatty acids by incorporating them into storage lipids (acyltransferases), and various enzymes that degrade fatty acids and storage lipids (e.g., lipases). The ability to tailor the lipid profile of a cell to increase the concentration of a particular fatty acid is desirable when targeting the lipid product to a specific market/application. Specifically, increasing the oleic acid content of an oleaginous yeast, like Yarrowia lipolytica, increases the value of the TAG produced in the organism. The lipid yield of oleaginous organisms can be increased by the up-regulation, down-regulation, or deletion of genes implicated in a lipid pathway. The successful modulation of enzymes, however, is unpredictable, at best.

For Microbial oil production, the most important is the natural ability of microorganisms to accumulate significant amounts of storage lipids in cells. Storage lipid accumulation in these so-called oleaginous microorganisms can occur in two ways. De novo lipid accumulation is connected with synthesis of fatty acids (FA) from various hydrophilic carbon substrates. The ability to accumulate lipids is connected with specific carbon metabolism, which enables oleaginous microorganisms to direct carbon towards FA synthesis. Redirecting of carbon towards FA synthesis is usually triggered by exhaustion of nitrogen. The other way is ex novo biosynthesis, and it is simply incorporation of extracellular FA into cells of oleaginous microorganism. It is not connected with nutrient depletion; the only limitation is the ability to transport FA into the cell. In recent years, many metabolic engineering tools have been used to study the lipid biosynthesis of microorganisms with the aim to improve lipid accumulation.

US20240026395A1 Disclosed transformed cells comprising one or more genetic modifications that affect the lipid content of the cell, e.g., by increasing the concentration of oleic acid in the cell relative to an unmodified cell of the same type. The document describes the multi-step genetic engineering step to modify the lipid content and a first genetic modification, wherein the first genetic modification comprises a knockout mutation of a native Δ12 desaturase protein; a second genetic modification, wherein the second genetic modification increases the expression of an elongase protein, a diacylglycerol acyltransferase protein, or a glycerol-3-phosphate acyltransferase protein; and a third genetic modification, wherein the third genetic modification comprises a nucleic acid encoding an exogenous fungal Δ9 desaturase protein or an exogenous Δ9 desaturase protein that comprises increased specificity for C18 a fatty acid relative to a native Δ9 desaturase protein of the modified yeast cell.

US11492647B2 Disclosed are methods and compositions for increasing the triacylglycerol content of a cell by increasing the activity of a type 1 diacylglycerol acyltransferase (i.e., DGA2) and increasing the activity of a type 2 diacylglycerol acyltransferase (i.e., DGA1). In some embodiments, the triacylglycerol content of a cell is also modified my decreasing the activity of a triacylglycerol lipase in the same cell. Also disclosed are methods and compositions for increasing the triacylglycerol content of a cell by increasing the activity of a type 1 diacylglycerol acyltransferase (i.e., DGA2), or by increasing the activity of a type 3 diacylglycerol acyltransferase (i.e., DGA3).

US10443047B2 Disclosed are methods and compositions for increasing the triacylglycerol content of a cell by up-regulating diacylglycerol acyltransferase and down-regulating triacylglycerol lipase. In some embodiments, a DGA1 protein is expressed and a native TGL3 gene is knocked out, thereby increasing the synthesis of triacylglycerol and decreasing its consumption, respectively.

US10760105B2 Disclosed are transformed cells comprising one or more genetic modifications that increase the lipid content of the cell, e.g., relative to an unmodified cell of the same type. Also disclosed are methods for increasing the lipid content of a cell by increasing the activity of one or more proteins in the cell and/or by decreasing the activity of one or more proteins in the cell.

US8951776B2 Some aspects of the present invention provide engineered microorganisms for oil production. Methods of microbial manipulation and manipulation of microorganisms are also provided herein. In some embodiments, a combination of rate-controlling steps of lipid synthesis, for example, a step (push step) of producing a metabolite for lipid synthesis, acetyl-CoA, ATP or NADPH, and feedback of lipid synthesis (Pull) step of isolating the product or intermediate of the lipid synthesis pathway mediating the inhibition of the activity of the microorganism. Such push-and-pull engineered microorganisms exhibit greatly improved conversion yield and TAG synthesis and storage properties.

US10920233B2 disclosed genetically modified yeast cell comprises (i) a recombinant acvl-CoA: diacvlglvcerol acvltransferase 1 (DGA1) gene and a genetically modified lipid synthesis regulator (MGA2) gene, wherein said genetically modified MGA2 gene increases lipid production compared to a non-genetically modified MGA2 control; or (ii) a recombinant acvl-CoA:diacvlglvcerol acvltransferase 2 (DGA2) gene and a genetically modified lipid synthesis regulator (MGA2) gene, wherein said genetically modified MGA2 gene increases lipid production compared to a non-genetically modified MGA2 control.

US11884940B2 discloses the present application relates to methods to improve biomass or lipid production in a microorganism from one or more fatty acid and one or more simple carbon co-substrates. Produced lipids may include unsaturated C6-C24 fatty acids, alcohols, aldehydes, and acetates which may be useful as final products or precursors to insect pheromones, fragrances, flavors, and polymer intermediates. The application further relates to recombinant microorganisms modified for improved production of biomass or lipid, or improved lipid selectivity. Also provided are methods of producing one or more lipid using the recombinant microorganisms, as well as compositions comprising the recombinant microorganisms and/or optionally one or more of the product lipids.

Ren et al., 2020 demonstrated the role of TiO2, TiC, SiC and g-C3N4 nanoparticles to study growth and lipid accumulation of Scenedesmus sp. (Bioresource Technology Volume 297, February 2020, 122409) and demonstrated lipids accumulation enhances in the presence of nanoparticles of microalgal cells.

He et al., 2017 disclosed Carbon nanotubes (CNTs), α-Fe₂O₃ nanoparticles (nano Fe₂O₃) and MgO nanoparticles (nano MgO) were evaluated for the effects on algae growth and lipid production. Nano Fe2O3 promoted cell growth in the range of 0-20 mg·L⁻¹. CNTs, nano Fe2O3 and nano MgO inhibited cell growth of Scenedesmus obliquus at 10, 40 and 0.8 mg·L⁻¹ respectively. Neutral lipid and total lipid content increased with the increasing concentration of all tested nanoparticles. The maximum lipid productivity of cultures exposed to CNTs, nano Fe2O3 and nano MgO was observed at 5 mg·L⁻¹, 5 mg·L⁻¹ and 40 mg·L⁻¹, with the improvement by 8.9%, 39.6% and 18.5%. High dose exposure to nanoparticles limited increase in lipid productivity, possibly due to the repression on cell growth caused by nanoparticles-catalyzed reactive oxygen species (ROS) generation, finally leading to reduction in biomass and lipid production. Reduced accumulation of fatty acids of C18:3n3, C18:3n6 and C20:2 was observed in cells exposed to nanoparticles.

Kolhe et al., 2022 overviewed the interaction of Yarrowia lipolytica and its interactions with Metals and demonstrated that Yarrowia lipoltycia in the presence of metals, levels of inherently produced metal binding proteins (metallothioneins) and the pigment melanin are seen to be elevated. Morphological alterations with respect to biofilm formation and dimorphic transition from yeast to mycelial form are also induced by certain metals. The biomass of Y. lipolytica is inherently important as a biosorbent and cell surface modification, process optimization or whole cell immobilization techniques have aided in improving this capability. In the presence of metals such as mercury, cadmium, copper and uranium, the culture forms nanoparticulate deposits. In addition, on account of its intrinsic reductive ability, Y. lipolytica is being exploited for synthesizing nanoparticles of gold, silver, cadmium and selenium with applications as antimicrobial compounds, location agents for bioimaging and as feed supplements. This versatile organism thus has great potential in interacting with various metals and addressing problems related to their pollutant status.

There are several processes to produce microbial oil using various microorganisms and described elsewhere. Most lipids synthesized by oleaginous microorganisms are of 4 to 22 unbranched carbon chain length. It can be saturated or unsaturated fatty acids depending on the nature of the hydro carbonated chain, while it can be monounsaturated or polyunsaturated fatty acids (MUFA and PUFA) depending on the number of double bonds. On the basis of the fatty acid profiles of oleaginous microorganisms, they can be utilized either for biodiesel production, biolubricants production or bio additives or for nutraceuticals.

However, the main challenge associated with oleaginous microorganisms is the secretion of the varied carbon chain length by varied microbial groups. For instance, microalgae, filamentous fungi, yeast, and bacteria produces a fatty acid rich in saturated and monounsaturated fatty acids (MUFA) content, whereas thraustochytrids and some genetically modified yeast, bacteria, and filamentous fungi produces microbial oil rich in polyunsaturated fatty acids (PUFA). Microalgal derived oil production faces significant drawback in large scale implementations due to the requirement of appropriate photobioreactors and low yield with rich in saturated fatty acids contents over 50% of the total oil composition. Whereas oleaginous yeast Yarrowia lipolytica can be cultivated in large scale fermenter system with fast growing and oil accumulation ability with less accumulation of saturated fatty acids and rich in MUFA content. For biolubricants or bio-additives application, the microbial derived oil should be of higher unbranched carbon chain length with less saturated fatty acids (SFA) content and carbon chain length of >C18 are desired. At present the majority of environmentally acceptable industrial lubricants (EAIL) are derived from plant-based vegetable oils, their application is limited due to poor thermal- oxidation stability and low temperature fluidity. The yeast derived microbial oil rich in MUFA or PUFA content with less SFA fraction offers an alternative solution to the plant derived oils and it improves the cold flow property with higher viscosity and suitable for base oil formulation and biolubricants applications.

Although microbial oil production was extensively studied for the past three decades across the globe, the challenges associated with their commercialization includes, low biomass growth, oil yield and lack of desired higher carbon chain length. Further genetic engineering intervention is attempted to improve the higher carbon chain length of the microbial oil, the tunability of the carbon chain length is not feasible due to the metabolic limitations and strict cultivation requirements for the genetically engineered microbes such as high-cost media requires which makes the process cumbersome.

### OBJECTIVES OF THE INVENTION

The main objective of the present invention is to provide a process for the preparation of tailor made microbial oil with higher unbranched carbon chain length MUFA/ PUFA content and less saturated fatty acids (SFA) content for application in biodiesel or biolubricants or bio-additives.

Another objective of the present invention is to provide a process for the preparation of tailor made microbial oil from organic rich feed stock or industrial byproduct or CO₂ derived intermediates.

### SUMMARY OF THE INVENTION

This summary is provided to introduce a selection of concepts, in a simplified format, that are further described in the detailed description of the invention. This summary is neither intended to identify key or essential inventive concepts of the invention and nor is it intended to determine the scope of the invention.

The present invention provides a process for the conversion of a waste feedstock into microbial oil, wherein the process comprises mixing the waste feedstock with wet microbial cells of a mutant yeast strain selected from a group consisting of Yarrowia lipolytica to obtain a culture media, wherein the mutant yeast strain has an optimum growth temperature in a range of 28 to 32 °C, in particular 30 °C; adding 0.01 % of simethicone antifoam agent to the culture media; sterilizing the culture media at 105 to 121 °C for 15 to 20 minutes, and augmenting a growth inducer in the culture media; incubating and fermenting the culture media obtained from preceding step for 72 hours to obtain a fermented media, wherein the mutant yeast strain is subjected to an intermittent temperature phase in the presence of the growth inducer under aerobic conditions to obtain the fermented media; wherein the growth inducer is present in a range of 0.01 to 10 weight % of the mutant yeast strain; separating a biomass from the fermented media; drying the biomass through heating method or spray drying method to obtain a dried cell biomass; crushing the dried cell biomass using mechanical grinder to obtain a dried cell biomass powder; and extracting the microbial oil from the dried cell biomass powder.

### DETAILED DESCRIPTION OF DRAWINGS

**Figure 1** illustrates the process for the conversion of waste feedstock into microbial oil using a mutant yeast strain in the presence of growth inducers on application of intermittent temperature phase.
**Figure 2** depicts the flow diagram for the process for the conversion of waste feedstock into microbial oil.

### DETAILED DESCRIPTION OF THE INVENTION

For the purpose of promoting an understanding of the principles of the invention, reference will now be made to the embodiments in the specific language to describe the same. It will nevertheless be understood that no limitation of the scope of the invention is thereby intended, such alterations and further modifications in the illustrated process, and such further applications of the principles of the invention as illustrated therein being contemplated as would normally occur to one skilled in the art to which the invention relates. Unless otherwise defined, all technical and scientific terms used herein have the same meaning as commonly understood by one of ordinary skilled in the art to which this invention belongs. The composition, methods, and examples provided herein are illustrative only and not intended to be limiting.

The articles "a", "an" and "the" are used to refer to one or to more than one (i.e., to at least one) of the grammatical object of the article.

The term "some" as used herein is defined as "none, or one, or more than one, or all". Accordingly, the terms "none", "one", "more than one", "more than one, but not all" or "all" would all fall under the definition of "some". The term "some embodiments" may refer to no embodiments or to one embodiment or to several embodiments or to all embodiments. Accordingly, the term "some embodiments" is defined as meaning "no embodiment, or one embodiment, or more than one embodiment, or all embodiments".

More specifically, any terms used herein such as but not limited to "includes", "comprises", "has", "consists" and grammatical variants thereof is inclusive or open-ended and does not exclude additional, unrecited elements or method steps. The specification will be understood to also include embodiments which have the transitional phrase "consisting of" or "consisting essentially of" in place of the transitional phrase "comprising". The transitional phrase "consisting of" excludes any element, step, or ingredient not specified in the claim, except for impurities associated therewith. The transitional phrase "consisting essentially of' limits the scope of a claim to the specified materials or steps "and those that do not materially affect the basic and novel characteristic(s)" of the claimed invention.

Whether or not a certain feature or element was limited to being used only once, either way it may still be referred to as "one or more features" or "one or more elements" or "at least one feature" or "at least one element". Furthermore, the use of the terms "one or more" or "at least one" feature or element do NOT preclude there being none of that feature or element, unless otherwise specified by limiting language such as "there NEEDS to be one or more" or "one or more element is REQUIRED".

Use of the phrases and/or terms such as but not limited to "a first embodiment", "a further embodiment", "an alternate embodiment", "one embodiment", "an embodiment", "multiple embodiments", "some embodiments", "other embodiments", "further embodiment", "furthermore embodiment", "additional embodiment" or variants thereof do NOT necessarily refer to the same embodiments. Unless otherwise specified, one or more particular features and/or elements described in connection with one or more embodiments may be found in one embodiment, or may be found in more than one embodiment, or may be found in all embodiments, or may be found in no embodiments. Although one or more features and/or elements may be described herein in the context of only a single embodiment, or alternatively in the context of more than one embodiment, or further alternatively in the context of all embodiments, the features and/or elements may instead be provided separately or in any appropriate combination or not at all. Conversely, any features and/or elements described in the context of separate embodiments may alternatively be realized as existing together in the context of a single embodiment.

The terminology and structure employed herein is for describing, teaching, and illuminating some embodiments and their specific features and elements and does not limit, restrict, or reduce the spirit and scope of the invention.

In order to overcome the earlier limitations, the present invention is envisaged to improve the oil content and tailor-made synthesis of microbial oil rich in MUFA/ PUFA content which have direct application in biodiesel or biolubricants or bio-additives.

The present invention provides process for the conversion of a waste feedstock into microbial oil, wherein the process comprises:
i. mixing the waste feedstock with wet microbial cells of a mutant yeast strain to obtain a culture media, wherein the mutant yeast strain has an optimum growth temperature in a range of 28 to 32 °C, in particular 30 °C;
ii. adding 0.01 % of simethicone antifoam agent to the culture media;
iii. sterilizing the culture media at 105 to 121 °C for 15 to 20 minutes, and augmenting a growth inducer in the culture media;
iv. incubating and fermenting the culture media obtained from step (iii) for 72 hours to obtain a fermented media, wherein the mutant yeast strain is subjected to an intermittent temperature phase in the presence of the growth inducer under aerobic conditions to obtain the fermented media; wherein the growth inducer is present in a range of 0.01 to 10 weight % of the mutant yeast strain.
v. separating a biomass from the fermented media;
vi. drying the biomass through heating method or spray drying method to obtain a dried cell biomass;
vii. crushing the dried cell biomass using mechanical grinder to obtain a dried cell biomass powder; and
viii. extracting the microbial oil from the dried cell biomass powder.

In an embodiment of the present invention, the wet microbial cells of the mutant yeast strain are obtained by a process comprises:
i. growing a mutant yeast strain in a YPD (yeast peptone and dextrose) growth media at a temperature in arrange of 25-35 °C with a stirring speed in a range of 80-120 rpm till optical density of the growth media reaches within a range of 3 to 5; and
ii. centrifuging the YPD growth media of step (i) to obtain the wet microbial cells of the mutant yeast strain.

In an embodiment of the present invention, the intermittent temperature phase comprises raising or lowering of the growth temperature of the mutant yeast strain in a range of 15 to 55 °C.

In an embodiment of the present invention, the mutant yeast strain is exposed to the intermittent temperature phase between 0 to 1500 minutes in particular 0 to 60 minutes, 0 to 30 minutes, 0 to 15 minutes, and 0 to 10 minutes.

The process of the present invention involves the addition of growth inducers and providing intermittent temperature-shock to induce the biomass yield and oil accumulation. The mutated yeast used herein improves the synthesis trait of tailor-made chain length and process conditions herein to improve the production yield of biomass and oil quality. The mutated yeast was subject to process optimization with varied temperature-shock conditions and the presence of growth inducers. The intermittent temperature shock conditions of raising or lowering the growth temperature of yeast 28-32 °C (growth temperature) in a range of 15 to 55 °C along with the growth inducers in a range of 0.1 to 1% of the yeast DCW enhanced the oil accumulation and biomass growth. The produced microbial oil has potential application for biodiesel, biolubricants and bio-additives.

In an embodiment of the present invention, the mutant yeast strain is selected from a group consisting of Yarrowia lipolytica. In a preferred embodiment, the mutant yeast strain is Yarrowia lipolytica W29.

In a preferred embodiment of the process, microbial oil production can be operated in batch or continuous mode of operation. The oxygen requirement of the process can be in the range of 20 to 100%, preferably about 50% of the dissolved oxygen (DO) requirement for oil accumulation. The oxygen can be passed through the culture medium through any suitable device forming fine dispersion of gas result in an increase in contact area. Further, oxygen gas can be purged via micro-bubbler or nano-bubbler system.

In an embodiment of the present invention, the growth inducers comprise:
i. oxides of vanadium, molybdenum, antimony, niobium
ii. at least one element selected from the group consisting of lithium, titanium, tin, iron, manganese, nickel, cobalt, copper, zinc.
iii. a bio-compatible metal catalyst capable of inducing oxygen stress at ambient conditions, wherein the bio-compatible metal catalyst is a monometallic bio-compatible catalyst or a bimetallic bio-compatible catalyst. The yeast mutant strain is grown on a mixed metal oxide nanoparticle selected from at least one of transition metals series, alumina, zirconia, zeolites, and a combination thereof.

In a preferred embodiment of the present invention, there is provided a process wherein growth inducer is selected from a transition metals series is Titanium oxide. The size of the nanoparticles used herein was in the range of 10 to 150 nm. The desired concentration of titanium oxide nanoparticles was in the range of 0.01 to 10 % of the total yeast cell weight. In still another embodiment, the other nanoparticles can be chosen from Nickel, Iron, Zinc, Magnesium, Cobalt, Copper alone or in combination thereof. In another embodiment of the process, the nanomaterial of Fe, Ni, Zn, Co, Cu, Mg, and Ti were synthesized and added into the fermentation media in a range of 0.01 to 10 % after the sterilization process to prevent the agglomeration of the nanoparticles and the media components during heating process. The mutant strain was evaluated for their biomass growth, oil content, and FAME content after 72 hours of incubation under aerobic conditions. The growth temperature was maintained at 24 to 36 ° C under shaking condition of 100 rpm in an incubator shaker.

In an embodiment of the present invention, the waste feedstock is an organic rich feed stock, an industrial byproduct, and CO₂ derived intermediates; and wherein the culture media comprises a nitrogen source, a carbon source, inorganic salts and other nutrients.

The mutant yeast strain described herein can withstand the temperature of 25 to 50 °C and can grow in the presence of inducer and are able to accumulate oil inside the cell biomass. The mutant yeast strain is cultivated in a culture medium containing carbon source as glucose, molasses, glycerol, acetate, and press mud and other conventional nutrients such as nitrogen source and inorganic salts. Examples of suitable nitrogen sources include organic and inorganic nitrogen rich compounds such as peptone, yeast extract, urea, ammonium chloride, ammonium nitrate, ammonium sulphate, ammonium sulfate, and ammonium phosphate. Examples of inorganic salts include salts of phosphates, sulfates and hydrochlorides of sodium, potassium, magnesium, iron, nickel, Zinc, V, Titanium, Copper, cobalt, such as KH₂PO₄, K₂HPO₄, NaCl, MgSO₄.7H₂O, FeSO₄.7H₂O, NiCl₂·6H₂O, ZnSO₄.7H₂O, V₂O₅, TiO₂ , CuCl₂, and CoCl₂. The other nutrients such as yeast extract, malt extract, and D-biotin, are added to the medium to assist the yeast growth.

In another embodiment of the present invention, the culture media comprises a nitrogen base without amino acids and ammonium sulfate in a range of 1.7 to 3.4 g/L, NH₄Cl in a range of 0.5 to 2.5 g/L, phosphate buffer having a pH 6.8, and a carbon source in a range of 10 to 100 g/L selected from a group comprises glucose, acetic acid, butyric acid, molasses, glycerol, acetate, and press mud.

In another embodiment of the present invention, a process of inducing the physiological stress of the mutant yeast via exposure of the actively grown cell to various intermittent temperature conditions. In a preferred embodiment, the intermittent decreased temperature of 20 °C for a period of 60 minutes. In another embodiment, the intermittent increased temperature was 45 °C for a period of 15 minutes. In still another embodiment, the intermittent temperature variation can be tested either by increasing or decreasing the optimal growth conditions of mutant strain (25 to 32 °C ). The increment can be in the order of (35, 37, 40, 45 or 50 ° C), whereas the lower temperature can be in the order of (5,10,15, 17, and 20 ° C). The exposure time of the mutant strain with intermittent temperature phase can be varied between 0 to 1500 minutes, or in particular 0 to 60 minutes or 0 to 10 minutes. After the expose of the intermittent change in the temperature, the growth temperature of the mutant yeast strain was maintained at 28 to 32 °C.

In another embodiment of the present invention, the mutant yeast strain can be exposed to both oxidative and physiological stress conditions. In a preferred embodiment, the yeast cell exposed to an intermittent high temperature of 45 °C for a period of 15 minutes, followed by Ti nanoparticle addition triggered the oil accumulation and biomass growth.

In an embodiment of the present invention, the biomass is separated from the fermented media through centrifugation or sedimentation of fermentation media using organic and inorganic coagulants selected from a group comprising ferric chloride, potassium aluminum sulfate (PAS), polyaluminum chloride (PAC), and chitosan.

In an embodiment of the present invention, the biomass is dried in a heating furnace at a temperature of 60 to105 °C for a period of 4 to 6 hours.

In an embodiment of the present invention, the microbial oil is extracted from the dried cell biomass powder by adding a solvent mixture comprises chloroform and methanol to the dried cell biomass powder to obtain a biomass mixture; separating lipids from the biomass mixture through vortex and centrifugation of the mixture; adding hexane to the separated lipids to obtain the washed lipids; and evaporating hexane from the washed lipids to obtain the purified lipids comprising the microbial oils.

In an embodiment of the present invention, the dried cell biomass powder to the solvent mixture has a ratio of 1:60 w/v.

In an embodiment of the present invention, the biomass mixture is vortexed for 10 to 20 minutes, in particular 15 minutes and centrifugation of biomass mixture is carried out at 10000 to 15000 rpm for 10 to 20 minutes, in particular for 13000 rpm for 15 minutes.

The present invention also provides a process for producing mutant yeast strain of *Yarrowia lipolytica,* wherein the process comprises:
i. inoculating the mutant yeast strain of *Yarrowia lipolytica* into a fresh medium to obtain microbial cells, wherein the microbial cells have an optical density of 1.8 at 600nm;
ii. centrifuging the microbial cells at 8000 rpm for 10 minutes;
iii. washing the centrifuged microbial cells obtained from preceding step twice with 0.1 M sodium phosphate buffer having a pH 6.0 and resuspending the washed microbial cells in 5 ml sterile saline water of 0.9% (wt/vol) at a concentration of 3.0×10¹¹ cells ml⁻¹ to obtain a cell suspension;
iv. irradiating 400 µl of the cell suspension to UV rays (235 nm) at a distance of 20 cm for 5 minutes;
v. diluting the irradiated cell suspension of preceding step by 107-fold; and
vi. adding 100 µl samples of the diluted cell suspensions in YPD media and allowing cultivation for 48 hours at 29 °C to obtain the mutant yeast strain.

In an embodiment of the present invention, the fresh medium comprises yeast extract (10.0 g/L), peptone (20.0 g/L), dextrose (20.0 g/L), and optionally agar 20.0 g/L, and the fresh medium has a pH 6.5+/-0.2.

In an embodiment of the present invention, the mutant yeast strain is further subjected to N-methyl-N'-nitro-N-nitrosoguanidine (NTG) treatment comprises:
i. preparing a log growth phase of mutant yeast strain in YPD media, wherein the log growth phase has an optical density of 1.3 at 600nm;
ii. adding 9 ml of the log growth phase to 1 ml of a sterile solution containing 1 to 10 mg/ml NTG, in particular 3 mg/mL in phosphate buffer;
iii. shaking the solution of preceding step at 29 to 32 °C, in particular at 29 °C for 30 minutes;
iv. centrifuging the solution for 10 minutes at 8,000 rpm to obtain the NTG treated mutant yeast strain; and
v. washing the NTG treated mutant yeast strain three times with sterile water and resuspended in 10 ml of sterile phosphate buffer having a pH 7.2.

In another embodiment of the present invention, the dried cells were used for oil extraction and fatty acid methyl ester composition.

In an embodiment of the present invention, total lipids were estimated using the Folch method, which involves chloroform and methanol (2:1, v/v) solvent mixture in a ratio of 1:60 (biomass: solvent mixture, w/v). For this, 50 mg of dried biomass powder was added to 15 mL screw-cap tubes containing 3 mL of the solvent mixture. This mixture was vortexed for 5 minutes, followed by centrifugation at 13,000 rpm for 15 minutes. The process was repeated three times to ensure complete lipid extraction. Hexane was added to the tube, and the upper layer was carefully collected using a Pasteur pipette. The solvent was then evaporated under a nitrogen stream, and the lipid content was determined gravimetrically.

For FAME analysis, Dry biomass powder (50 mg) was added to 15 mL screw-cap tubes containing a 3 mL solvent mixture of methanol or ethanol, hexane, and concentrated HCl in a 10:1:1 ratio (v/v/v). The tubes were incubated under inert atmosphere at 90 °C for 3 h at 300 rpm, followed by cooling with running water to stop the reaction. One milliliter of hexane was added in to the tube and vortexed for 2 minutes followed by addition of 1 mL of water (repeated twice). Water was added into tube for clear hexane phase separation. The upper hexane layers were carefully collected with a Pasteur pipette, and solvent evaporation was performed under a nitrogen stream. Fatty acid methyl esters (FAME) or fatty acid ethyl esters (FAEE) were estimated using gas chromatography.

### EXAMPLES:

The present disclosure with reference to the accompanying examples describes the present invention. However, those of skill in the art should, in light of the present disclosure, appreciate that many changes can be made in the specific embodiments which are disclosed and still obtain a like or similar result without departing from the spirit and scope of the invention. It is understood that the examples are provided for the purpose of illustrating the invention only and are not intended to limit the scope of the invention in any way.

### Example 1 Development of mutant strain

In an embodiment of the present invention, there is provided a process wherein the mutant yeast strain is selected from the group consisting of Yarrowia lipolytica (ATCC 20460). In another embodiment of the present invention, there is provided a process wherein the mutant yeast strain is obtained from Yarrowia lipolytica W29 (ATCC 20460).

### Development of Mutant of Yarrowia lipolytica (ATCC 20460) and its Characterization

### (a) Characteristics of Wild strain Yarrowia lipolytica (ATCC 20460)

The wild strain Yarrowia lipolytica ( ATCC 20460) belongs to fungi classification and was obtained from American Type Culture Collection (ATCC). The typical growth media for the wild strain includes yeast extract (10.0 g/L), peptone (20.0 g/L), dextrose (20.0 g/L), agar (if required) 20.0 g/L, pH 6.5+/-0.2. The typical temperature for growth was between 24° C to 32° C under aerobic conditions.

### (b) Mutagenesis Protocol of Yarrowia lipolytica (ATCC 20460)

The wild strain of Yarrowia lipolytica (ATCC 20460) was inoculated into fresh medium yeast extract (10.0 g/L), peptone (20.0 g/L), dextrose (20.0 g/L), agar (if required) 20.0 g/L, pH 6.5+/-0.2). Cells at optical density 1.8 at 600nm were centrifuged at 8000 rpm for 10 minutes, washed twice with 0.1 M sodium phosphate buffer (pH 6.0), and were resuspended in a tube with 5 ml sterile saline water of 0.9% (wt/vol) at a concentration of 3.0×1011 cells ml⁻¹. Further, 400 µl of the above cell suspension was placed in a sterile petri dish and exposed to UV rays (235 nm) at a distance of 20 cm. After a duration of 5 minutes, the irradiated cell samples were collected, diluted 107-fold, and 100-µl samples of each of the diluted suspensions were plated on YPD medium. After about 48 hours of cultivation at 29° C., all the individual colonies on each plate with approximately 15 individual colonies were inoculated on slants.

The colonies from the slants were tested for the cell capacity to survive in 100 ml YPD broth, and the wild strain was used as a control culture under the same conditions except the wild culture was not irradiated. Based on the highest OD at 600 nm (OD as a measure of biomass accumulation with YPD medium), one strain with highest growth rate was selected.

The obtained strain was further subjected to N-methyl-N'-nitro-N-nitrosoguanidine (NTG) treatment. For NTG-treatment, a Log growth phase of strain in YPD media was prepared (OD: 1.3 at 600 nm). 9 ml of which was added to 1 ml of a sterile solution of NTG (3 mg/ml NTG in phosphate buffer; solution freshly prepared lhour before use). The samples were shaken at 29 °C. for 30 minutes and immediately centrifuged for 10 minutes at 8,000 rpm, and the supernatant was decanted. The cells were washed three times with sterile water and resuspended in 10 ml of sterile phosphate buffer (pH 7.2). All the experimental samples were serially diluted with sterile water and plated on YPD plates. The YPD plates were grown for 72 hours at 29 °C. 20 morphologically different colonies were selected and investigated for oil production using acetate as feed in YPD medium. Finally, the mutants that produce the highest oil yield within 72 hours were considered positive mutants because of its higher oil production capacity than that of the wild strain.

**TABLE 1 Physiological properties of wild and mutant strain**

| **Temperature** | **Wild** | **Mutant** |
|---|---|---|
| 25 | + | + |
| 27 | + | + |
| 29 | + | + |
| 32 | + | + |
| 37 | + | + |
| 45 | - | + |
| 47 | - | + |

### Example 2: Bio process to produce oil from mutant strain Yarrowia lipoltyica W29.

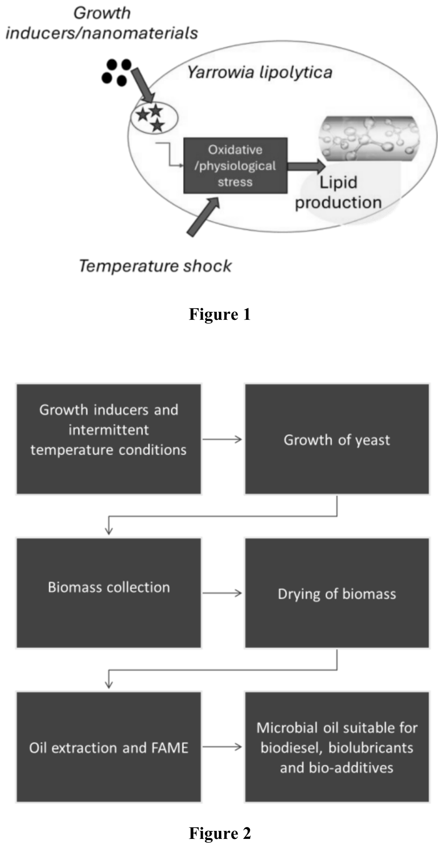

Initially, the enriched mutant strain was grown in a YPD agar plate, the selectively grown individual colony was picked and transferred in a sterile YPD broth for the cultivation of mutant. The mutant was grown in an incubator shaker at a temperature of 30 °C and a speed of 100 rpm was maintained for sufficient mixing. The media used for oil production used herein was prepared using the following ingredients Yeast Nitrogen Base without amino acids and ammonium sulfate;1.7 to 3.4 g/L, NH₄Cl: 0.5 to 2.5 g/L, phosphate buffer (pH 6.8), carbon source (glucose) 10 to 100 g/L. Simethicone antifoam agent of 0.01% was used. The oil production media was sterilized at 105 °C to 121 °C for 15 to 20 minutes, after cooling down to room temperature used for yeast cultivation. 10% over night grown cultured in YPD broth was used as an inoculum source, followed by incubation for a period of 72 hours. After the fermentation period of 72 hours, the cells were harvested and tested for their biomass accumulation and oil production. The process for the preparation of microbial oil is depicted in Figure 1 and Figure 2.

**Table 2 Biomass and oil production data of the wild and mutant strain with glucose as feedstock**

| **Microbes** | **Biomass Yield** | **Oil Content %** | **Fame Composition** | | | | | |
|---|---|---|---|---|---|---|---|---|
| | | | **C 16** | **C 18** | **C 18.1** | **C 18.2** | **C 18.3** | **Others** |
| Wild type | 3.5 g/L | 16 % | 42.2 | 9.4 | 27.1 | 18 | 2.3 | 0.5 |
| Mutant-1 | 5.4 g/L | 28 % | 32.8 | 1.2 | 14.1 | 31.5 | 16.7 | 3.7 |
| Mutant-2 | 4.5 g/L | 26 % | 31.4 | 9.2 | 12.7 | 33.1 | 10.6 | 3.0 |

### Example 3: Performance of mutant yeast on CO₂ derived volatile fatty acids

The enriched mutant was selected and grown overnight in YPD media and evaluated for their growth and oil production in the presence of CO₂ derived waste materials. In a separate process, the Syngas and CO₂ fermented by anaerobic bacteria to form a mixture of VFAs, typically enriched with acetic acid and butyric acid or the VFA can be obtained from anaerobic digestion plant effluent or dark fermentation process. Further, the growth of Yarrowia lipolytica W29 mutant strain and oil production ability on acetic acid and butyric was demonstrated and presented herein. The media used for lipid accumulation comprises Nitrogen Base without amino acids and ammonium sulfate;1.7 to 3.4 g/L, NH₄Cl: 0.5 to 2.5 g/L, phosphate buffer (pH 6.8), carbon source (acetic acid, butyric acid) 10 to 100 g/L alone or in combination were used. Simethicone antifoam agent of 0.01% was used. The mutant strain was evaluated for their biomass growth, oil content, and FAME content after 72 hours of incubation under aerobic conditions. The growth temperature was maintained at 24 to 36 °C under shaking condition of 100 rpm in an incubator shaker.

**Table 3: Bio oil production from CO₂ derived volatile fatty acids.**

| **Feed** | **Biomass Yield** | **Oil Content** | **Fame Composition** | | | | | |
|---|---|---|---|---|---|---|---|---|
| | | | **C 16** | **C 18** | **C 18.1** | **C 18.2** | **C 18.3** | **Others** |
| Acetic acid | 2.5 | 35 | 23.7 | 0.5 | 9.4 | 42.6 | 15.2 | 8.7 |
| Butyric acid | 2.1 | 36 | 31.4 | 2.9 | 2.7 | 35.1 | 15.6 | 12.3 |
| Acetic acid and Butyric acid 25:75 mixture | 2.2 | 42 | 19.0 | 16 | 46.4 | 23.5 | 8.9 | 1.0 |
| Acetic acid and Butyric acid 50:50 mixture | 2.6 | 32 | 21.6 | 1.5 | 42.9 | 23.8 | 7.5 | 2.7 |
| Acetic acid and Butyric acid 75:25 mixture | 2.6 | 33 | 28.3 | 1.5 | 37.3 | 24.1 | 4.7 | 3.7 |

### Example 4: Bioprocess of bio-oil production from mutant strain with various feedstock

In another embodiment of the present invention, the process described herein used various feedstock such as glucose, molasses, glycerol. Acetate and press mud for biomass growth and oil accumulation of the mutant strain of Yarrowia lipolytica W29.

The media used for oil production used herein was prepared using the following ingredients Yeast Nitrogen Base without amino acids and ammonium sulfate;1.7 to 3.4 g/L, NH₄Cl: 0.5 to 2.5 g/L, phosphate buffer (pH 6.8), carbon source (glucose, molasses, glycerol, acetate, and press mud) 10 to 100 g/L. Simethicone antifoam agent of 0.01% was used. The oil production media was sterilized at 105 °C to 121 °C for 15 to 20 minutes, after cool down to room temperature used for yeast cultivation. 10 % over night grown cultured in YPD broth was used as an inoculum source, followed by incubation for a period of 72 hours. After the fermentation period of 72 hours, the cells were harvested and tested for their biomass accumulation and oil production. The biomass accumulation was quantified using dry cell weight of the biomass. For dry cell weight estimation, the desired volume of fermentation broth was harvested using a centrifuge at a speed of 8000 rpm for 10 mins, followed by two-times washing with distilled or ultrapure water to remove the impurities, the wet slurry was dried at 105 °C for 4 to 6 hours or until the wait becomes constant. The oil content of yeast was obtained via drying the yeast in an oven at 105 °C for 4 to 6 hours and the dried cells were used for oil extraction and fatty acid methyl ester composition. In brief, Total lipids were estimated using the Folch method, which involves chloroform and methanol (2:1, v/v) solvent mixture in a ratio of 1:60 (biomass: solvent mixture, w/v). For this, 50 mg of dried biomass powder was added to 15 mL screw-cap tubes containing 3 mL of the solvent mixture. This mixture was vortexed for 5 minutes, followed by centrifugation at 13,000 rpm for 15 minutes. The process was repeated three times to ensure complete lipid extraction. Hexane was added to the tube, and the upper layer was carefully collected using a Pasteur pipette. The solvent was then evaporated under a nitrogen stream, and the lipid content was determined gravimetrically.

For FAME analysis, Dry biomass powder (50 mg) was added to 15 mL screw-cap tubes containing a 3 mL solvent mixture of methanol or ethanol, hexane, and concentrated HCl in a 10:1:1 ratio (v/v/v). The tubes were incubated under inert atmosphere at 90 °C for 3 h at 300 rpm, followed by cooling with running water to stop the reaction. One milliliter of hexane was added in to the tube and vortexed for 2 minutes followed by addition of 1 mL of water (repeated twice). Water was added into tube for clear hexane phase separation. The upper hexane layers were carefully collected with a Pasteur pipette, and solvent evaporation was performed under a nitrogen stream. Fatty acid methyl esters (FAME) or fatty acid ethyl esters (FAEE) were estimated using gas chromatography. Table 4 summarized the biomass and oil content of the mutant strain across various feedstock.

**Table 4: Bio-oil production from various feedstocks using mutant strain.**

| **Feed** | **Biomass yield** | **Oil Content** | **Fame Composition** | | | | | |
|---|---|---|---|---|---|---|---|---|
| | | | **C 16** | **C 18** | **C 18.1** | **C 18.2** | **C 18.3** | **Others** |
| Glucose | 5.4 | 28 | 42.2 | 9.4 | 2.3 | 27.1 | 18 | 0.5 |
| Molasses | 7.8 | 38 | 39.8 | 2.4 | 4.5 | 32.9 | 16.5 | 3.9 |
| Glycerol | 3.8 | 42 | 44.8 | 2.1 | 8.7 | 22.8 | 16.5 | 5.1 |
| Acetate | 2.5 | 35 | 23.7 | 0.5 | 9.4 | 42.6 | 15.2 | 8.7 |
| Press Mud | 2.0 | 38 | 67.3 | 0.2 | 6.5 | 15.7 | 4.7 | 5.6 |

### Example 5: Performance of the mutant in the presence of growth inducers

The enriched mutant was selected and grown overnight in YPD media and evaluated for their growth and oil production in the presence of growth inducers. In a separate process, the nanomaterial of Fe, Ni, Zn, Co, Cu, Mg, and Ti were synthesized and added into the fermentation media at a rate of 0.01 to 10 % after the sterilization process to prevent the agglomeration of the nanoparticles and the media components during heating process. The media used for lipid accumulation comprises Nitrogen Base without amino acids and ammonium sulfate; 1.7 to 3.4 g/L, NH₄Cl: 0.5 to 2.5 g/L, phosphate buffer (pH 6.8), carbon source (molasses) 10 to 100 g/L. Simethicone antifoam agent of 0.01% was used. The mutant strain was evaluated for their biomass growth, oil content, and FAME content after 72 hours of incubation under aerobic conditions. The growth temperature was maintained at 24 to 36 °C under shaking condition of 100 rpm in an incubator shaker.

Table 5 represents the biomass yield, oil content, and FAME yield of the mutant strain in the presence of various growth inducers. It has been found that the product titer and oil content value significantly increase in the presence of Ti nanomaterials at a desired dosage of 0.01 to 10% concentration. The increase in oil accumulation and biomass yield implies that Ti nanomaterials induces the metabolism of the yeast cell.

### Preparation of inducer:

### The inducers of were prepared as per the following method:

Fe, Ni, Zn, Co, Mg, Ti, and Cu, metal salts of sulfate or nitrate, reducing agents such as sodium borohydride (NaBH4), and stabilizers like polyvinylpyrrolidone (PVP) or citrate. Solven: distilled water or ethanol
a. Dissolve1.0 grams of the metal salt in 50 mL of distilled water to create a homogeneous solution. Adjust the concentration depending on the desired nanoparticle size. To control nanoparticle growth and prevent aggregation, add 0.1 to 1.0 grams of stabilizer to the solution. With the solution stirring at a moderate speed, slowly add the reducing agent, typically 0.05 to 0.5 grams of sodium borohydride, to initiate the reduction process. The reaction can occur at room temperature or with mild heating (30-50 °C).
b. Maintain consistent stirring for 30 to 60 minutes to ensure thorough reduction and uniform nanoparticle formation. As the reduction proceeds, you should notice a change in color, indicating successful nanoparticle formation. Once the reaction is complete, separate the nanoparticles from the solution using a centrifuge set at 5,000 to 10,000 rpm for 10 to 20 minutes. This will create a solid pellet at the bottom of the centrifuge tube.
c. Remove the supernatant and wash the pellet with distilled water or ethanol to eliminate residual chemicals. Repeat the centrifugation and washing process 2 to 3 times to ensure purity.

**Table 5: performance of mutant strain in the presence of growth inducers**

| **Microbes** | **Biomass yield** | **Oil Content** | **Fame Composition** | | | | | |
|---|---|---|---|---|---|---|---|---|
| | | | **C 16** | **C 18** | **C 18.1** | **C 18.2** | **C 18.3** | **Others** |
| Mutant | 7.8 | 38 | 39.8 | 2.4 | 4.5 | 32.9 | 16.5 | 3.9 |
| Fe | 8.2 | 39 | 31.8 | 11.1 | 16.5 | 29.4 | 9.8 | 0.4 |
| Ni | 9.8 | 41 | 27.9 | 8 | 38.8 | 11.6 | 9.6 | 4.1 |
| Zn | 8.9 | 44 | 30.3 | 5.1 | 33.6 | 14.7 | 11.7 | 4.6 |
| Co | 7.2 | 39 | 27.2 | 7.7 | 34.7 | 16.4 | 10.6 | 3.4 |
| Mg | 8.8 | 45 | 37.9 | 12.8 | 32.3 | 17 | 5.5 | 2.1 |
| Ti | 10.5 | 42 | 30.4 | 9.4 | 28.3 | 21 | 8.6 | 1 |
| Cu | 7.8 | 41 | 48.1 | 4.4 | 23.6 | 3.8 | 7.1 | 13.1 |

### Example 6: Behaviour of mutant strain under Physiological stress conditions Intermittent temperature shock.

The mutant strain was further evaluated under various intermittent temperature shock conditions to further assess their oil production ability. The media used for lipid accumulation comprises Nitrogen Base without amino acids and ammonium sulfate; 1.7 to 3.4 g/L, NH₄Cl: 0.5 to 2.5 g/L, phosphate buffer (pH 6.8), carbon source (molasses) 10 to 100 g/L. Simethicone antifoam agent of 0.01% was used. The mutant strain was evaluated for their biomass growth, oil content, and FAME content after 72 hours of incubation under aerobic conditions. The intermittent temperature variation can be tested either by increasing or decreasing the optimal growth conditions of mutant strain (25 to 32 °C). The increment can be in the order of (35, 40, 45, 47 or 50 °C), whereas the lower temperature can be in the order of (5,10,15, 17, and 20 °C). The exposure time of the mutant strain with intermittent temperature phase can be varied between 0 to 720 minutes, or in particular 0 to 60 minutes or 0 to 30 minutes or 0 to 15 minutes.

Table 6 demonstrates the selectivity of the biomass yield and oil accumulation of the mutant strain varied with the physiological stress conditions. Exposure of the mutant strain to a low intermittent temperature showed a least biomass production, however the C18.1 content was enriched in the total FAME, whereas the biomass growth and oil content was significant enhanced with the presence of high temperature conditions, the oil yield of 45% was attained under high intermittent temperature phase conditions.

**Table 6: Growth of mutant strain under intermittent temperature phase physiological stress conditions.**

| **Physiological stress condition** | **Biomass yield** | **Oil Content** | **Fame Composition** | | | | | |
|---|---|---|---|---|---|---|---|---|
| | | | **C 16** | **C 18** | **C 18.1** | **C 18.2** | **C 18.3** | **Others** |
| Mutant strain as such | 7.8 | 38 | 39.8 | 2.4 | 4.5 | 32.9 | 16.5 | 3.9 |
| Low temperature shock -15 °C for 30 min | 6.8 | 41 | 34.1 | 8.3 | 16.7 | 35.7 | 4.2 | 1 |
| Low temperature shock -17 °C for 30 min | 7.2 | 38 | 33.5 | 3.8 | 18 | 35.8 | 7.8 | 1.1 |
| Low temperature shock -20 °C for 30 min | 7.3 | 40 | 32.5 | 2.6 | 17.5 | 36.8 | 6.5 | 4.1 |
| Low temperature shock -35 °C for 30 min | 8.1 | 38 | 33.8 | 2.8 | 16.8 | 37.8 | 8.5 | 0.3 |
| Low temperature shock -40 °C for 30 min | 8.8 | 42 | 34.8 | 1.8 | 19 | 35.8 | 7.9 | 0.7 |
| Low temperature shock -45 °C for 30 min | 9.1 | 42 | 26.8 | 2.8 | 19.2 | 43.5 | 3.8 | 3.9 |
| Low temperature shock -47 °C for 30 min | 9.5 | 45 | 28.5 | 2.5 | 18.5 | 41.5 | 7.8 | 1.2 |
| Low temperature shock -50 °C for 30 min | 7.2 | 38 | 26.8 | 1.8 | 20.5 | 35.8 | 14.8 | 0.3 |

### Example 7: Growth behavior of mutant strain in the combined stress conditions of growth inducers and physiological stress

The mutant strain was further evaluated under presence of growth inducer and intermittent temperature shock conditions to further assess their oil production ability. The media used for lipid accumulation comprises Nitrogen Base without amino acids and ammonium sulfate;1.7 to 3.4 g/L, NH₄Cl: 0.5 to 2.5 g/L, phosphate buffer (pH 6.8), carbon source (molasses) 10 to 100 g/L. Simethicone antifoam agent of 0.01% was used. The mutant strain was evaluated for their biomass growth, oil content, and FAME content after 72 hours of incubation under aerobic conditions.

**Table 7: Growth behavior of yeast under combined stress conditions.**

| **Stress Condition** | **Biomass yield** | **Oil Content** | **Fame Composition** | | | | | |
|---|---|---|---|---|---|---|---|---|
| | | | **C 16** | **C 18** | **C 18.1** | **C 18.2** | **C 18.3** | **Others** |
| Mutant strain as such | 7.8 | 38 | 39.8 | 2.4 | 4.5 | 32.9 | 16.5 | 3.9 |
| Intermittent high temperature -47 °C for 30 min | 9.5 | 45 | 28.5 | 2.5 | 18.5 | 41.5 | 7.8 | 1.2 |
| Low temperature shock -15 °C for 30 min | 6.8 | 41 | 34.1 | 8.3 | 16.7 | 35.7 | 4.2 | 1 |
| Ti Nanoparticle | 10.5 | 42 | 30.4 | 9.4 | 28.3 | 21 | 8.6 | 1 |
| Intermittent high temperature + Ti nanoparticle | 11.2 | 47 | 32.8 | 1.2 | 14.1 | 31.5 | 16.7 | 3.7 |
| Intermittent low temperature + Ti nanoparticle | 10.8 | 48 | 31.4 | 2.9 | 12.7 | 35.1 | 15.6 | 2.3 |

Table 7 demonstrates the ability of the mutant strain to grow in the presence of both oxidative (growth inducer, Ti-nanomaterial) and physiological stress conditions. As demonstrated, the mutant strain can survive under both harsh conditions and improves the oil production titer and quality. Under both stress conditions the yeast cell accumulates oil inside the cells and results in improved the oil content of the yeast. A maximum oil yield of about 47% was obtained under stress conditions, whereas the native strain attained 18% of total oil yield.

### Example 8: Tailor made synthesis of microbial oil under intermittent temperature and growth inducer dosing

The mutant strain was further evaluated under intermittent temperature shock conditions and growth inducer dosing at different time intervals to further assess their oil production ability. The media used for lipid accumulation comprises Nitrogen Base without amino acids and ammonium sulfate;1.7 to 3.4 g/L, NH4Cl: 0.5 to 2.5 g/L, phosphate buffer (pH 6.8), carbon source (molasses) 10 to 100 g/L. Simethicone antifoam agent of 0.01% was used. The mutant strain was evaluated for their biomass growth, oil content, and FAME content after 72 hours of incubation under aerobic conditions.

**Table 8 Tailor made synthesis of microbial oil under intermittent temperature and growth inducer dosing.**

| **Stress condition** | **Biomass yield** | **Oil Content** | **Fame Composition** | | | | | |
|---|---|---|---|---|---|---|---|---|
| | | | **C 16** | **C 18** | **C 18.1** | **C 18.2** | **C 18.3** | **Others** |
| Mutant strain as such | 7.8 | 38 | 39.8 | 2.4 | 4.5 | 32.9 | 16.5 | 3.9 |
| Intermittent high temperature -47 °C for 30 min after 12 h of incubation | 10.1 | 45 | 32.5 | 2.3 | 12.5 | 41.5 | 9.8 | 0.4 |
| Intermittent high temperature -47 °C for 30 min after 24 h of incubation | 8.9 | 43 | 33.8 | 6.3 | 3.7 | 45 | 9.6 | 1.6 |
| Intermittent high temperature -47 °C for 30 min after 48 h of incubation | 8.1 | 42 | 38.9 | 10.8 | 7.8 | 23.5 | 18.6 | 0.4 |
| Intermittent high temperature -47 °C for 30 min + Ti nanoparticle dosing | 11.2 | 52 | 32.8 | 1.2 | 14.1 | 31.5 | 16.7 | 3.7 |
| Intermittent high temperature -47 °C for 30 min after 12 hour of incubation + Ti nanoparticle dosing | 12.2 | 55 | 24.9 | 2.4 | 12.8 | 42 | 17.5 | 0.4 |
| Intermittent high temperature -47 °C for 30 min after 24 hour of incubation + Ti nanoparticle dosing | 11.8 | 51 | 32.1 | 3.5 | 12.7 | 35.1 | 16.2 | 0.4 |
| Intermittent high temperature -47 °C for 30 min after 48 hour of incubation + Ti nanoparticle dosing | 10.8 | 48 | 33 | 2.8 | 11.5 | 33.8 | 15.2 | 3.7 |

Table 8 demonstrates the tailor-made synthesis of microbial oil under combined intermittent temperature and growth inducers conditions. Intermittent temperature (47 °C for 30 minutes) changes during the growth phase conditions of 12h, 24 h, and 48 h applied to the mutant strain along with growth inducers. The maximum biomass growth (12.2 g/L) and oil yield of about 55% was obtained under the combined conditions of intermittent temperature exposure of 47 °C for 30 minutes after 12 h of incubation and Ti nanoparticles additions. The FAME content rich in PUFA over 59.5 %, MUFA 12.8 %, and SFA content of 27.3% were also obtained. This clearly, indicates that the yeast strain can be fine-tuned for oil accumulation using the mutant strategy, the mutant strain shows improvement in production of oil and tailor-made carbon chain length derivatives during the physiological/oxidative stress conditions, the produced oil may have direct application in biodiesel or base oil/ bio-based additives.

## Claims

1. A process for the conversion of a waste feedstock into microbial oil, wherein the process comprises:
i. mixing the waste feedstock with wet microbial cells of a mutant yeast strain to obtain a culture media, wherein the mutant yeast strain has an optimum growth temperature in a range of 28 to 32 °C, in particular 30 °C;
ii. adding 0.01 % of simethicone antifoam agent to the culture media;
iii. sterilizing the culture media at 105 to 121 °C for 15 to 20 minutes, and augmenting a growth inducer in the culture media;
iv. incubating and fermenting the culture media obtained from step (iii) for 72 hours to obtain a fermented media, wherein the mutant yeast strain is subjected to an intermittent temperature phase in the presence of the growth inducer under aerobic conditions to obtain the fermented media; wherein the growth inducer is present in a range of 0.01 to 10 weight % of the mutant yeast strain.
v. separating a biomass from the fermented media;
vi. drying the biomass through heating method or spray drying method to obtain a dried cell biomass;
vii. crushing the dried cell biomass using mechanical grinder to obtain a dried cell biomass powder; and
viii. extracting the microbial oil from the dried cell biomass powder.

2. The process as claimed in claim 1, wherein the wet microbial cells of the mutant yeast strain are obtained by a process comprises:
i. growing a mutant yeast strain in a YPD (yeast peptone and dextrose) growth media at a temperature in arrange of 25-35 °C with a stirring speed in a range of 80-120 rpm till optical density of the growth media reaches within a range of 3 to 5; and
ii. centrifuging the YPD growth media of step (i) to obtain the wet microbial cells of the mutant yeast strain.

3. The process as claimed in claim 1, wherein the intermittent temperature phase comprises raising or lowering of the growth temperature of the mutant yeast strain in a range of 15 to 55 °C.

4. The process as claimed in claim 1, wherein the mutant yeast strain is exposed to the intermittent temperature phase between 0 to 1500 minutes in particular 0 to 60 minutes, 0 to 30 minutes, 0 to 15 minutes, and 0 to 10 minutes.

5. The process as claimed in claims 1-4, wherein the mutant yeast strain is selected from a group of consisting of Yarrowia lipolytica, in particular the mutant yeast strain is Yarrowia lipolytica W29.

6. The process as claimed in claim 1, wherein the mutant yeast strain is subjected to the intermittent temperature phase in a batch mode of operation or in a continuous mode of operation, wherein the process under aerobic conditions has an oxygen requirement in the range of 20 to 100%, preferably about 50% dissolved oxygen (DO) concentration.

7. The process as claimed in claim 1, wherein the growth inducer comprises at least one selected from:
i. oxides of vanadium, molybdenum, antimony and niobium;
ii. at least one element selected from the group consisting of lithium, titanium, tin, iron, manganese, nickel, cobalt, copper, and zinc; and
iii. a bio-compatible transition metal catalyst, wherein the bio-compatible metal catalyst is a monometallic bio-compatible catalyst or a bimetallic bio-compatible catalyst wherein the bio-compatible metal catalyst comprises mixed metal oxide nanoparticles selected from oxides of transition metals, alumina, zirconia, zeolites and a combination thereof and wherein the oxide of transition metal is titanium oxide nanoparticles, and wherein the nanoparticles have a particle size in a range of 10 to 150 nm.

8. The process as claimed in claim 1, wherein the waste feedstock is an organic rich feed stock, an industrial byproduct, and CO₂ derived intermediates; and wherein the culture media comprises a nitrogen source, a carbon source, inorganic salts and other nutrients wherein the carbon source is selected from a group comprising glucose, molasses, glycerol, acetate, acetic acid, butyric acid, and press mud, the nitrogen source comprises organic and inorganic nitrogen rich compounds selected from a group comprising peptone, yeast extract, urea, ammonium chloride, ammonium nitrate, ammonium sulphate, ammonium sulfate, and ammonium phosphate, the inorganic salts comprises salts of phosphates, sulfates, hydrochlorides of sodium, potassium, magnesium, iron, nickel, zinc, vanadium, titanium, copper, and cobalt, selected from a group comprising KH₂PO₄, K₂HPO₄, NaCl, MgSO₄.7H₂O, FeSO₄.7H₂O, NiCl₂·6H₂O, ZnSO₄.7H₂O, V₂O₅, TiO₂, CuCl₂, and CoCl₂; the other nutrients comprises yeast extract, malt extract, and D-biotin and wherein the culture media comprises a nitrogen base without amino acids and ammonium sulfate in a range of 1.7 to 3.4 g/L, NH₄Cl in a range of 0.5 to 2.5 g/L, phosphate buffer having a pH 6.8, and a carbon source in a range of 10 to 100 g/L selected from a group comprises glucose, acetic acid, butyric acid, molasses, glycerol, acetate, and press mud.

9. The process as claimed in claim 1, wherein the biomass is separated from the fermented media through centrifugation or sedimentation of fermentation media using organic and inorganic coagulants selected from a group comprising ferric chloride, potassium aluminum sulfate (PAS), polyaluminum chloride (PAC), and chitosan.

10. The process as claimed in claim 1, wherein the biomass is dried in a heating furnace at a temperature of 60 to 105 °C for a period of 4 to 6 hours.

11. The process as claimed in claim 1, wherein the microbial oil is extracted from the dried cell biomass powder by adding a solvent mixture comprises chloroform and methanol to the dried cell biomass powder to obtain a biomass mixture; separating lipids from the biomass mixture through vortex and centrifugation of the mixture; adding hexane to the separated lipids to obtain the washed lipids; and evaporating hexane from the washed lipids to obtain the purified lipids comprising the microbial oils wherein the dried cell biomass powder to the solvent mixture has a ratio of 1:60 w/v.

12. The process as claimed in claim 11, wherein the biomass mixture is vortexed for 10 to 20 minutes, in particular 15 minutes and centrifugation of biomass mixture is carried out at 10000 to 15000 rpm for 10 to 20 minutes, in particular for 13000 rpm for 15 minutes.

13. A process for producing mutant yeast strain of *Yarrowia lipolytica,* wherein the process comprises:
i. inoculating the mutant yeast strain of *Yarrowia lipolytica* into a fresh medium to obtain microbial cells, wherein the microbial cells have an optical density of 1.8 at 600nm;
ii. centrifuging the microbial cells at 8000 rpm for 10 minutes;
iii. washing the centrifuged microbial cells obtained from preceding step twice with 0.1 M sodium phosphate buffer having a pH 6.0 and resuspending the washed microbial cells in 5 ml sterile saline water of 0.9% (wt/vol) at a concentration of 3.0×10¹¹ cells ml⁻¹ to obtain a cell suspension;
iv. irradiating 400 µl of the cell suspension to UV rays (235 nm) at a distance of 20 cm for 5 minutes;
v. diluting the irradiated cell suspension of preceding step by 107-fold; and
vi. adding 100 µl samples of the diluted cell suspensions in YPD media and allowing cultivation for 48 hours at 29 °C to obtain the mutant yeast strain.

14. The process as claimed in claim 18, wherein the fresh medium comprises yeast extract (10.0 g/L), peptone (20.0 g/L), dextrose (20.0 g/L), and optionally agar 20.0 g/L, and the fresh medium has a pH 6.5+/-0.2.

15. The process as claimed in claim 18, wherein the mutant yeast strain is further subjected to N-methyl-N'-nitro-N-nitrosoguanidine (NTG) treatment comprises:
i. preparing a log growth phase of mutant yeast strain in YPD media, wherein the log growth phase has an optical density of 1.3 at 600nm;
ii. adding 9 ml of the log growth phase to 1 ml of a sterile solution containing 1 to 10 mg/ml NTG, in particular 3 mg/ml in phosphate buffer;
iii. shaking the solution of preceding step at 29 to 32 °C, in particular 29 °C for 30 minutes;
iv. centrifuging the solution for 10 minutes at 8,000 rpm to obtain the NTG treated mutant yeast strain; and
v. washing the NTG treated mutant yeast strain three times with sterile water and resuspended in 10 ml of sterile phosphate buffer having a pH 7.2.
